# EUROPEAN PATENT SPECIFICATION

(11) **EP 1 842 550 B1**
(45) Date of publication and mention of the grant of the patent: **16.01.2013**
(21) Application number: 07105399.5
(22) Date of filing: 25.03.2004
(51) Int. Cl.: A61K 45/00, A61K 31/593, A61K 31/616, A61K 38/19, A61K 38/48, A61K 39/39, A61K 39/35, A61K 38/17, A61P 11/06, A61P 37/08

(54) **Composition for inducing immunotolerance**
Zusammensetzungen zur Induktion von Immuntoleranz
Compositions pour induire une immunotolérance

(30) Priority: 28.03.2003 EP 03075909
(43) Date of publication of application: 10.10.2007
(62) Divisional of application: 04723429.9
(73) Proprietor: HAL Allergy Holding B.V., 2333 CH Leiden (NL)
(72) Inventor: van Oosterhout, Antonius Josephus Maria, 3573 PJ, Utrecht (NL); Kapsenberg, Martien Lukas, 1106 GW, Amsterdam (NL); Weller, Frank Reinoud, 1391 RK, Abcoude (NL); Taher, Yousef Al-Madane, 3706 AA, Zeist (NL); Lobato-van Esch, Elisabeth Catharina Adriana Maria, 3527 WH, Utrecht (NL); Vissers, Joost Lambert Max, 6525 XE, Nijmegen (NL)
(74) Representative: van Kooij, Adriaan

(56) References cited:
- WO-A-95/32734
- GB-A- 2 260 904
- ERB K J ET AL: "Novel vaccines protecting against the development of allergic disorders: a double-edged sword?" CURRENT OPINION IN IMMUNOLOGY, CURRENT BIOLOGY LTD, XX, vol. 14, no. 5, 1 October 2002 (2002-10-01), pages 633-643, XP004377420 ISSN: 0952-7915
- LARCHE M: "SPECIFIC IMMUNOTHERAPY" BRITISH MEDICAL BULLETIN, CHURCHILL LIVINGSTONE, LONDON, GB, vol. 56, no. 4, 2000, pages 1019-1036, XP001033878 ISSN: 0007-1420
- APPLEMAN LEONARD J ET AL: "Helper T cell anergy: From biochemistry to cancer pathophysiology and therapeutics" JOURNAL OF MOLECULAR MEDICINE, SPRINGER VERLAG, DE, vol. 78, no. 12, 2001, pages 673-683, XP002187721 ISSN: 0946-2716
- WONG C K ET AL: "Activation of p38 mitogen-activated protein kinase and nuclear factor-kappaB in tumour necrosis factor-induced eotaxin release of human eosinophils." CLINICAL AND EXPERIMENTAL IMMUNOLOGY, vol. 128, no. 3, June 2002 (2002-06), pages 483-489, XP001154027 June, 2002 ISSN: 0009-9104
- KANG S-M ET AL: "TRANSACTIVATION BY AP-1 IS A MOLECULAR TARGET OF T CELL CLONAL ANERGY" SCIENCE (WASHINGTON D C), vol. 257, no. 5073, 1992, pages 1134-1138, XP001154020 ISSN: 0036-8075
- JONULEIT H ET AL: "Dendritic cells as a tool to induce anergic and regulatory T cells" TRENDS IN IMMUNOLOGY, ELSEVIER, CAMBRIDGE, GB, vol. 22, no. 7, 1 July 2001 (2001-07-01), pages 394-400, XP004247295 ISSN: 1471-4906
- KIMATA M ET AL: "Effects of luteolin, quercetin and baicalein on immunoglobulin E-mediated mediator release from human cultured mast cells." CLINICAL AND EXPERIMENTAL ALLERGY, vol. 30, no. 4, April 2000 (2000-04), pages 501-508, XP001154026 ISSN: 0954-7894
- LI QIUTANG ET AL: "NF-kappaB regulation in the immune system." NATURE REVIEWS IMMUNOLOGY, vol. 2, no. 10, 20 October 2002 (2002-10-20), pages 725-734, XP009015224 ISSN: 1474-1733
- DAYNES RAYMOND A ET AL: "Emerging roles of PPARs in inflammation and immunity." NATURE REVIEWS IMMUNOLOGY, vol. 2, no. 10, 20 October 2002 (2002-10-20), pages 748-759, XP009015662 ISSN: 1474-1733
- D'AMBROSIO D ET AL: "INHIBITION OF IL-12 PRODUCTION BY 1,25-DIHYDROXYVITAMIN D3 INVOLVEMENT OF NF-KAPPAB DOWNREGULATION IN TRANSCRIPTIONAL REPRESSION OF THE P40 GENE" JOURNAL OF CLINICAL INVESTIGATION, AMERICAN SOCIETY FOR CLINICAL INVESTIGATION, US, vol. 101, no. 1, 1 January 1998 (1998-01-01), pages 252-262, XP001061567 ISSN: 0021-9738
- AMETAJ B N ET AL: "1,25-dihydroxyvitamin D-3 inhibits secretion of interferon-gamma by mitogen- and antigen-stimulated bovine mononuclear leukocytes" VETERINARY IMMUNOLOGY AND IMMUNOPATHOLOGY, AMSTERDAM, NL, vol. 52, no. 1-2, 1 January 1996 (1996-01-01), pages 77-90, XP002289076 ISSN: 0165-2427
- LEMIRE J M: "Immunomodulatory actions of 1,25-dihydroxyvitamin D-3" JOURNAL OF STEROID BIOCHEMISTRY AND MOLECULAR BIOLOGY, ELSEVIER SCIENCE LTD., OXFORD, GB, vol. 53, no. 1-6, 1 January 1995 (1995-01-01), pages 599-602, XP002289077 ISSN: 0960-0760
- MATHEU V ET AL: "DUAL EFFECTS OF VITAMIN D-INDUCED ALTERATION OF TH1/TH2 CYTOKINE EXPRESSION: ENHANCING IGE PRODUCTION AND DECREASING AIRWAY EOSINOPHILIA IN MURINE ALLERGIC AIRWAY DISEASE" JOURNAL OF ALLERGY AND CLINICAL IMMUNOLOGY, MOSBY - YEARLY BOOK, INC, US, vol. 112, no. 3, 1 September 2003 (2003-09-01), pages 585-592, XP009034088 ISSN: 0091-6749
- HEDLIN ET AL: "Immunotherapy in children with allergic asthma: Effect on bronchial hyperreactivity and pharmacotherapy" JOURNAL OF ALLERGY AND CLINICAL IMMUNOLOGY, MOSBY - YEARLY BOOK, INC, US, vol. 103, no. 4, 1 April 1999 (1999-04-01), pages 609-614, XP005687101 ISSN: 0091-6749
- LIEBERMAN PHIL: "Nonsteroidal anti-inflammatory drugs for the treatment of asthma and allergic disease" CURRENT CLINICAL PRACTICE; ALLERGIC DISEASES: DIAGNOSIS AND TREATMENT HUMANA PRESS INC. {A}, SUITE 808, 999 RIVERVIEW DRIVE, TOTOWA, NEW JERSEY 07512, USA, 1997, pages 315-322, XP009107758 ISSN: 0-89603-367-8
- KUMAR P ET AL: "ALLERGIC RHINITIS RELIEVED BY ASPIRIN AND OTHER NONSTEROIDAL ANTIINFLAMMATORY DRUGS" ANNALS OF ALLERGY, vol. 60, no. 5, 1988, pages 419-422, XP009107754 ISSN: 0003-4738
- SOMWAR ROMEL ET AL: "A dominant-negative p38 MAPK mutant and novel selective inhibitors of p38 MAPK reduce insulin-stimulated glucose uptake in 3T3-L1 adipocytes without affecting GLUT4 translocation." JOURNAL OF BIOLOGICAL CHEMISTRY, vol. 277, no. 52, 27 December 2002 (2002-12-27), pages 50386-50395, XP002501714 ISSN: 0021-9258

## Description

The invention relates to the induction of tolerance against an allergen, specifically to the immunisation with allergen and inhibiting the production of co-stimulator molecules in antigen presenting cells. The invention provides compositions for use in methods of treating allergic disorders.

### Adaptive immunity and peripheral tolerance

Adaptive immunity is initiated by the antigen-specific stimulation of naive T cells by peptide MHC class I or II complexes expressed by antigen-presenting cells (APCs, i.e. dendritic cells, macrophages, monocytes, B-lymphocytes). Effective responses, however, require the additional stimulation of naive T cells by "co-stimulator" molecules expressed by these APCs (Baxter *et al.,* 2002; Matzinger, 2002). The expression of "co-stimulator" molecules is part of the innate immune response induced by biologically active environmental substances (pathogen or any biologically active compound, including allergen) that affect migratory non-specific immune cells and/or resident tissue cells. The expression in APC is induced directly by the biological activity of environmental substances and/or indirectly by the reactivity products (stress and inflammatory mediators, necrotic cells) generated in response to these substances by other cells within the APC tissue micro-environment (Grallucci *et al.,* 2001).

The reactivity of non-specific immune cells, such as APCs, and resident cells to these biologically active compounds is part of the innate immune response and triggered in most if not all cases via the NF-κB and/or the MAPK/AP-1 signal transduction pathways (. Innate immunity activation pathways are triggered by compounds including but not limited to:
1. "Pathogen-associated molecular patterns" (PAMPs) present in endotoxins, peptidoglycans, carbohydrates and other microbial constituents by so-called "pattern recognition receptors" (PRR) including the Toll-like receptors, scavenger receptors and lectin receptors (Pulendran *et al.,* 2001; Reis e Sousa, 2001). PRR recognize not only exogenous molecules but also endogenous molecules such as heat-shock proteins and hyaluronan.
2. Mediators of oxidative stress generated within the APC microenvironment.
3. Heat-shock proteins affecting APCs through interaction with different cell-membrane receptors such as CD91 and toll-like receptors -2 and -4 (Gallucci *et al.,* 2001).
4. Intracellular nucleotides like ATP and UTP (Gallucci *et al.,* 2001).
5. Proteolytic enzymes that are locally released from resident cells or provided by the antigen itself and may activate these transcription factors through the protease-activated receptor family. In this respect, it is important to note that many allergens have proteolytic activities (Gallucci *et al.,* 2001). Moreover, allergens may also directly activate NF-κB in airway epithelial cells and allergen challenge rapidly activates NF-κB in airway epithelium in an animal model of asthma.
6. The family of cytosolic pattern recognition receptors for intracellular pathogens called nucleotide-binding oligomerization domain (NOD), also called caspase recruitment domain (CARD).

The NF-κB family of transcription factors and the transcription factor AP-1 have a central role in coordinating the expression of a wide variety of genes that control immune and inflammatory responses, including cytokines, chemokines, cell-adhesion molecules, co-stimulatory molecules, complement factors and anti-apoptotic factors (Herlaar *et al.,* 1999; McKay *et al.,* 1999). Whereas these molecules are central in the innate immune responses, they also initiate and tailor adaptive immune responses to these compounds by stimulating APC, in particular dendritic cells (DC), to express "co-stimulation" molecules that effectively alarm naïve T cells. DC reside in peripheral tissues as highly endocytic immature cells with low expression of co-stimulatory molecules. Activation of immature DC by exposure to certain environmental or stress compounds induces their maturation into mature DC with high cell surface expression of MHC molecules complexed with peptides from proteins that have been internalised in their immature stage, and high cell surface expression of "co-stimulation" molecules. Therefore, mature DC efficiently activate naive T cells specific against peptide-derived proteins from the environmental compounds.

Mature DC promote the development of subsets of immunogenic (such as Th1 or Th2) and/or tolerogenic (such as the regulatory cells Treg, Tr1 or Th3) T-cells, but the balance of these subsets strongly depends on the way the mature DC have been activated in their immature stage. Since different pathogens activate immature DC in different ways resulting in different functional phenotypes of mature DC, DC can tailor the class of specific immune response to the invading pathogen. Ideally, this process results in protection against this pathogen by immunogenic T cells without lethal pathology to host tissue induced by the activity of these Th cells. The selective development of T cell subsets is directed by the selective expression levels of cell-surface molecules such as co-stimulatory molecules (CD40, B7-family proteins and others) and by the production of T-cell skewing cytokines (IL-12, type 1 IFNs, IL-10, TGF-β and others).

Most importantly, in steady state conditions, in the absence of environmental biologically active compounds or stress reactions, T cells continuously engage immature DC with low expression "co-stimulation" molecules. Activation of Th cells in the absence of "co-stimulation" also results in the development of regulatory T cells that mediate tolerance to auto-antigens ubiquitously carried by these DC, another level of protection against autoimmunity.

Peripheral tolerance can be defined as the failure to respond to an antigen by an adaptive immune response and is acquired by mature lymphocytes in peripheral tissues. Although still incompletely understood, the mechanisms of action of peripheral tolerance can be due to (i) anergy, (ii) immune deviation, (iii) activation-induced cell-death (apoptosis) or other at present unknown mechanisms. Recently, regulatory T-lymphocytes have been shown to mediate peripheral tolerance in at least some immunological disease models such as colitis, transplant rejection, allergic- and auto-immune diseases. The family of regulatory T cells is diverse. They are all anergic, i.e. do not proliferate in response to antigen, and are tolerogenic, i.e. suppress the activity of immunogenic T cells. In non-pathogenic conditions Treg are thymus-derived, they suppress immunogenic Th cells via a cell-contact-dependent mechanism and are important in prevention of autoimmunity. Tr1 cells are characterized by the production of IL-10 and have been shown to suppress both Th1 and Th2 responses and thereby prevents the development of auto-immunity and allergic diseases. Th3 cells are characterized by the production of TGFβ and have been shown to be involved in oral tolerance.

The mechanism by which APCs, in particular DCs, induce the development of regulatory T cells is largely unknown. Although DC are involved in the generation of these forms of peripheral tolerance, it is at present unknown whether they are a different subset or are generated out of immature DC by (unknown) micro-environmental factors. DC that generate Tr1 cells have been characterized by the production of IL-10 whereas DC that generate Th3 cells have been characterized by the production of TGFβ. Antigen-specific regulatory T-cells producing IL-10 and/or anergic T-cells can also be generated by repetitive stimulation with immature DC that present antigen (Dhodapkar *et al.,* 2001; Jonuleit *et al.,* 2001; Roncarolo *et al.,* 2001). *In vitro* experiments suggest that suppression by the regulatory T cells induced by partially matured DC is cell-contact dependent (Jonuleit *et al.,* 2001).

### Allergen vaccination

Although allergen vaccination has been practiced since 1911, recent developments in purification of extracts and in understanding of the mechanism has increased its applicability at present and its promise for the future in the treatment of allergic diseases. Subcutaneous injection with increasing doses of allergen leads in the majority of allergic patients to reduction of allergen-induced inflammation, in a significant reduction in allergic symptoms and medication requirement and improvement in lung function as has been summarized in a meta-analysis. The clinical and anti-inflammatory (skin, conjunctivae) effects lasts even years after stopping the vaccination schedule.

Although this classical form of allergen vaccination is clearly beneficial for the treatment of mono-allergic asthma patients, it seldom results in complete alleviation of all symptoms. Moreover, occurrence of side effects at high doses, especially in asthma, the cumbersome application by repetitious injections during long periods of time and the strong clinical effects of concurrent therapies such as inhaled corticosteroids discourage physicians to use it at a large scale. Thus, there is strong need for novel strategies that improve allergen vaccination and reduce unwanted side effects.

### Mechanisms of allergen vaccination in allergic patients

The clinical effect of allergen vaccination (also called allergen-specific immunotherapy or immunotherapy) is likely to be mediated through reduction of allergen-induced inflammation. The immunological process underlying this effect remains at present unknown. As T-cells regulate the inflammatory response much effort has been focused on activation and differentiation of T-lymphocytes in relation to allergen vaccination. Both allergen specific hypo-reactivity, as a shift in the cytokine profile of the reacting T-lymphocytes (Th2 to Th1) have been proposed. This has opened the possibility to improve the efficacy of allergen vaccination by immunoregulatory cytokines such as IL-12 or IL-18. Recently, a potential role of IL-10 in the beneficial effect of allergen vaccination in bee-venom allergic patients has been demonstrated. During Bee-venom immunotherapy, the reduction of T-cell proliferation and cytokine responses (IL-5, IL-13) upon restimulation *in vitro* could be fully antagonized by neutralization of IL-10. These data suggest a role for IL-10 producing regulatory T-cells (Trl) in allergen vaccination. IL-10 has been shown to decrease IgE production and to enhance IgG4 production in human B-lymphocytes *in vitro.* At the T-cell level, IL-10 reduces T-cell responses to specific antigens by suppressing co-stimulatory signals (B7-1 and B7-2) delivered by antigen presenting cells.

### Pre-clinical model of allergic asthma in the mouse

Previously, we have developed a highly reproducible model in the mouse (BALB/c) with immunological and pathophysiological features reminiscent of allergic asthma e.g. antigen-specific IgE, eosinophilic airway inflammation and airway hyper-responsiveness to methacholine. These asthma features are associated with the appearance of Th2 cells in lung tissue and the draining lymph nodes. Said Th2 cells and the cytokines they produce, play a central role in the initiation and progression of the airway manifestations of asthma as shown by studies using monoclonal antibodies to cytokines and by depletion or transfer of T cell subsets.

### Allergen vaccination in α mouse model

We were the first to demonstrate that allergen vaccination is effective in a mouse model of allergic asthma. A protocol of subcutaneous allergen injections resembling a semi-rush protocol used in humans was effective to prevent allergen-induced airway hyperreactivity to methacholine and eosinophilic airway inflammation. During allergen vaccination, an initial rise in serum IgE levels occurred, after which IgE levels decreased sharply concomitant with an increase in IgG2a levels. The increase in IgG2a antibodies indicates a role for IFNy produced by either Th1-cells or Tr1 cells. The down-regulation of these airway manifestations of asthma was associated with decreased Th2 type cytokine production (IL-4 and IL-5) upon *in* vitro restimulation. These data suggest that the beneficial effect of allergen vaccination is mediated by an effect on Th2-lymphocytes such as (i) anergy, (ii) induction of Th1 or Th3/Tr1 cells ("immune deviation"), (iii) activation-induced cell-death (apoptosis) or other at present unknown mechanisms.

The present invention provides compositions for use in methods of treating allergic disorders as defined in the appended claims. The methods comprise administering di-hydroxyvitamin D3 compounds with administering an allergen. Said medicaments decrease the activity of APCs in such a way that the APC is still handling the antigen and exposes the epitopes on its surface to the lymphocyte, but the production of co-stimulator molecules is decreased or prevented. Therefore, the present invention teaches a method to induce and/or increase tolerance to an allergen in a subject, comprising inhibiting and/or preventing the production of a co-stimulator molecule in an antigen-presenting-cell in the presence of an allergen.

The allergen is administered to a person in need of such tolerance induction or enhancement. Because the co-stimulator molecules are expressed after triggering of the NF-κB and/or the MAPK/AP-1 signal transducing pathways, it is an object of the present invention to inhibit said pathways in APCs.

Said di-hydroxyvitamin D3 compounds are combined with one or more allergens. Therefore, the present invention teaches a pharmaceutical composition comprising di-hydroxyvitamin D3 compounds and one or more allergens, further comprising a suitable diluent. Said di-hydroxyvitamin D3 compounds may be administrated to a patient in need of such treatment before the administration of the allergens. The di-hydroxyvitamin D3 compounds may be administered via another route than said allergens. For example, the di-hydroxyvitamin D3 compounds may be provided orally, or topically, followed by topical administration of the allergens. Said topical administration comprises administration on the skin, and/or on the mucosa of the airways and/or of the oro-nasal cavity, and/or of the gastro-intestinal mucosa.

In another embodiment said di-hydroxyvitamin D3 compounds may be combined with said allergens before administration to a patient. Therefore, the present invention also discloses a pharmaceutical composition as mentioned above wherein said di-hydroxyvitamin D3 compounds is combined with said allergen before administration to a patient. Administration of abovementioned pharmaceutical compositions increases the induction of tolerance to allergens and may diminish disease symptoms in patients suffering from hypersensitivity to various allergens. Therefore, disclosed is a method to increase induction of immunotolerance, comprising providing a pharmaceutical composition as mentioned above by oral, and/or enteral, and/or intranasal, and/or dermal administration.

### Inhibition of NF-κB signal transduction pathway

The NF-κB family of transcription factors has a central role in coordinating the expression of a wide variety of genes that control immune- and inflammatory responses, including cytokines, chemokines, cell-adhesion molecules, co-stimulatory molecules, complement factors and anti-apoptotic factors (McKay *et al.,* 1999). Mammalian NF-κB family members include RelA (p65), NF-κB1 (p50; p150), NF-κB2 (p52; p100), cRel and RelB. Importantly, experiments with gene-deleted mice have proved that NF-κB1 p50, RelA and cRel are essential in the innate immune function of DC. NF-κB proteins are present in the cytoplasm in association with inhibitory proteins that are known as inhibitors of NF-κB (IκBs). The IκB family of proteins consists of IκBα, IκBβ, IκBε and BCL-3 (Li, NRDD). An essential step in the activation of innate immune cells by pathogens, stress molecules and pro-inflammatory cytokines is the degradation of IκB and release of NF-κB and its subsequent phosphorylation allowing NF-κB proteins to translocate to the nucleus and bind to their cognate DNA binding sites to regulate the transcription of large numbers of genes. A crucial regulatory step in the degradation of IκBs is the signal-induced phosphorylation of IκB at specific amino-terminal serine residues, which is mediated by serine-specific IκB kinases (IKK). The serine phosphorylated IκB is then ubiquitinilated and degraded by the proteasome. The IKK complex consists of several proteins, the main ones being IKK1 (IKKγ), IKK2 and the regulatory subunit NF-κB essential modulator (NEMO, also known as IKKγ).

Inhibition of NF-κB activation can be accomplished by several strategies including but not limited to direct targeting the DNA-bindang activity of individual NF-κB proteins using small molecules or decoy oligonucleotides; treatment with cell-membrane permeable non-degradable IκBα, -β or -ε mutant protein(s); blocking the nuclear translocation of NF-κB dimers by inhibiting the nuclear import system; stabilizing IκBα, -β or -ε protein(s) by developing ubiquitylation and proteasome inhibitors; and targeting signalling kinases such as IKK using small-molecule inhibitors (Li et *al.,* 2002); treatment with cell-membrane permeable dominant negative IKK protein. Several drugs that are used to treat inflammatory diseases have effects on NF-κB activity such as glucocorticosteroids (GCS), aspirin and other anti-inflammatory drugs. Although these drugs do not target NF-κB specifically, parts of their pharmacologic effects are due to inhibition of NF-κB activity. Besides these drugs, many compounds have been described in literature as inhibitors of NF-κB activation such as for example anti-oxidants, proteasome and protease inhibitors, κB phosphorylation and/or degradation inhibitors and miscellaneous inhibitors (table 1).

### Inhibition of MAPK/AP-1 signal transduction pathway

Mammals express at least four distinctly regulated groups of mitogen-activated protein kinases (MAPKs), ERK-1/2, ERK5, JNK1/2/3 and p38α/β/γ/δ, that have been shown to regulate several physiological and pathological cellular phenomena, including inflammation, apoptotic cell death, oncogenic transformation, tumor cell invasion and metastasis; Herlaar *et al.,* 1999). Upon cellular stimulation, a kinase cascade is initiated that ultimately leads to altered gene expression and consequently a biological response. The three main kinases in the MAPK cascades are the MAPK kinase kinase (MKKK), MAPK kinase (MKK) and MAPK. In total, 12 MAPK isoforms have been identified which can phosphorylate and activate a large range of substrates, including transcription factors and kinases. p38MAPK and JNK are stress-activated protein kinases that mediate responses to cellular stress factors such as UV light and oxidative stress. A wide variety of inflammatory mediators, such as cytokines, activate p38 MAPK in immune- and inflammatory cells. To date, several specific MAPK inhibitors have been developed in particular targeting p38 MAPK . The pyridinylimidazole compounds, exemplified by SB 203580, have been demonstrated to be selective inhibitors of p38 MAPK. This compound specifically inhibits p38α,β and β2 MAPK and has shown activity in a variety of animal models of acute and chronic inflammation. Other small molecule compounds that inhibit p38 MAPK are VX-745 (Vertex Pharmaceuticals), RWJ67657 (Johnson & Johnson) and HEP 689 (Leo Pharmaceuticals). Interestingly, SB 203580 has been shown to inhibit the maturation of dendritic cells. Other compounds that have been shown to inhibit dendritic cell maturation through inhibition of p38 MAPK are the anti-inflammatory sesquiterpene lactone parthenolide (PTL) and the cytokine IL-10. In contrast, inhibition of the ERK MAPK pathway by the selective inhibitors PD98059 and U0126, has been shown to enhance phenotypic and functional maturation of dendritic cells.

Little is known about the role of JNK MAPK in the regulation of innate- and adaptive immune responses. The JNK inhibitor SP600125 has been shown to inhibit the induction of IL-18 production by macrophages and the signalling of the T1/ST2, a cell-membrane receptor that is selectively expressed on Th2 lymphocytes.

MAPKs are upstream regulators of AP-1. The transcription factor family activator protein 1 (AP-1) is formed by heterodimeric complexes of a Fos protein (c-Fos, Fra-1, Fra-2, FosB and FosB2) with a Jun protein (c-Jun, JunB and JunD) or a homodimer between two Jun proteins (Foletta *et al.,* 1998). AP-1 regulates many of the genes up-regulated during immune- and inflammatory responses. The most well known repressor of the transcription factor AP-1 are glucocorticoids. Together with the inhibition NF-κB activation by glucocorticoids, these are the major mechanisms for the anti-inflammatory effects of this drug class (McKay *et al.,* 1999). Activation of gene transcription by AP-1 can also be inhibited by decoy oligonucleotides.

### Indirect inhibition of NF-κB and MAPK/AP-1 signal transduction pathways

The activation of the NF-κB and/or MAPK/AP-1 pathways can also be prevented by interference with "co-stimulation" molecules or locally produced activating mediators by (i) blocking of NF-κB and/or MAPK/AP-1 activating mediators, including but not limited to cytokines such as IL-1, -2, -12, -15, -17, -18, LIF, and members of the TNF super-family such as FAS ligand, GITR ligand, THANK, RANK ligand (also called TRANCE or OPGL), TNFα and TNFβ or blocking their specific cell-membrane receptors or (ii) blocking PRR including but not limited to toll-like receptors, lectin receptors or NODs or (iii) prevention of oxidative stress using anti-oxidants or (iv) blocking extra-cellular heat-shock proteins or their cell-membrane receptors or (v) blocking purinergic receptors, in particular those expressed on APCs.

NF-κB activation, in particular in APCs, can also be inhibited by compounds that increase intracellular levels of cyclic AMP including but not limited to β 2-adrenoceptor agonists, prostanoid EP2- or DP receptor agonists or phosphodiesterase IV inhibitors.

### Inhibition of NF-κB and MAPK/AP-1 signal transduction pathways by PPAR activation

Recently, an interesting family of nuclear hormone receptors have emerged called peroxisome proliferator-activated receptors (PPARs) which upon ligation exert potent inhibitory effects on the transcription factors NF-κB and AP-1 ( *Daynes et al.,* 2002; Hihi *et al.,* 2002). So far, three PPAR isoforms have been identified PPARα, PPARβ/δ and PPAR γ with a high degree of sequence and structural homology. PPARs share the property of forming heterodimers with another nuclear receptor of the same subgroup, the 9-cis-retinoic acid receptor (RXR) which appears to be essential for their biological function. Various types of fatty acids and eicosanoids can bind to and activate PPARs, with some degree of isoform specificity Daynes *et al.,* 2002; Hihi *et al.,* 2002). PPARα can be activated by α-linoleic-, γ-linoleic-, arachidonic- and eicosapentaenoic acids and by medium-chain saturated and monounsaturated fatty acids such as palmitic- and oleic acids. PPARα can be activated selectively by LTB4 and 8(S)HETE. PPARγ is activated by α-linoleic-, γ-linoleic-, arachidonic- and eicosapentaenoic acids although these endogenous ligands are weak activators. PPARγ is best stimulated by 9-HODE, 13-HODE and 15dPGJ2 and by the synthetic compound rosiglitazone and thiazolidinedione class of drugs. PPARβ/δ can be activated by some saturated, monounsaturated and unsaturated fatty acids, and by various eicosanoids including PGA1 and PGD2 and prostacyclin or a stable synthetic form. Interestingly, PPARα and PPARγ are expressed in antigen-presenting cells (monocytes, macrophages, dendritic cells and B-cells) and can play an important role in down-regulation of NF-κB and AP-1 activity (Daynes *et al.,* 2002; Nencioni *et al.,* 2002).

### DNA vaccination

The standard DNA vaccine consists of the specific gene(s) of interest cloned into a bacterial plasmid engineered for optimal expression in eukaryotic cells. Essential features include a strong promoter for optimal expression in mammalian cells, an origin of replication allowing growth in bacteria, a bacterial antibiotic resistance gene and incorporation of polyadenylation sequences to stabilize mRNA transcripts. Moreover, DNA vaccines also contain specific nucleotide sequences that play a critical role in the immunogenicity of these vaccines. In case of allergen vaccination using DNA vaccines, the plasmid contains nucleotide sequences encoding one or more allergens or allergen fragments containing at least one T-cell epitope sequence. Allergens for use in the invention include, but are not limited to the list available on the World-Wide Web at http://www.allergen.org/List.htm. It has been shown that immune responses induced by DNA vaccination are mediated by APCs, in particular DCs migrating from the site of vaccination to the draining lymph nodes. The DCs are either directly transfected or take up secreted protein from other transfected cells i.e. myocytes. Fusion of the allergen or allergen fragment to an IgG Fc fragment improves the secretion of the encoding allergen and the subsequent targeting to and uptake by APCs. Targeting of DNA vaccines to APCs, in particular DCs, may be obtained by using particular viral vectors including but not limited to herpes virus, vaccinia virus, adenovirus, influenza virus, retroviruses and lentiviruses (Jenne et al., 2001). Second-generation DNA vaccines are also being developed that introduce not only a gene encoding the target antigen, but also a gene encoding some other factor capable of inducing an altered immune response. Within the present invention, the plasmid comprising a T-cell epitope can be combined with genes encoding proteins that inhibit the activation of the NF-κB pathway, including but not limited to (non-degradable) IxB proteins or dominant negative forms of IKK proteins or NF-κB proteins and/or genes encoding proteins that inhibit the activation of the MAPK/AP-1 pathway, including but not limited to dominant negative forms of critical proteins leading to the activation of Fos and/or Jun proteins such as p38 MAPK or dominant negative forms of Fos and/or Jun proteins. In another embodiment, the plasmid comprising a T-cell epitope can be combined with small interfering RNA sequences or anti-sense sequences that inhibit the expression of IKK, NF-κB, p38 MAPK or AP-1 proteins.

Of course, the effects of inhibiting or preventing the production of a co-stimulator molecule in an antigen-presenting-cell, when said antigen presenting cell is contacted with an allergen, can also be studied and assessed on isolated cells *in vitro.* The effects of a compound on the production of a co-stimulator molecule can thus be tested *in vitro* and a selection can be made as to what compound is most suitable for inhibiting the production of a co-stimulator molecule by an antigen presenting cells. Therefore, disclosed is a method to inhibit and/or prevent the production of a co-stimulator molecule in an antigen-presenting-cell in the presence of an allergen, wherein said production of a co-stimulator molecule is inhibited and/or prevented by inhibiting the NF-κB and/or the MAPK/AP-1 signal transducing pathways in said antigen-presenting-cell.

### Novel allergen vaccination strategies

In the most recent WHO position paper, allergen vaccination or immunotherapy is defined as the practice of administering gradually increasing quantities of an allergen extract to an allergic subject to ameliorate the symptoms associated with subsequent exposure to the causative allergen (Bousquet *et al.,* 1998). In the present invention, we describe novel forms of allergen vaccination that offer significant advantages over current allergen immunotherapy practice.

Allergens for use in the invention include, but are not limited to the list available on the World-Wide Web at http://www.allergen.org/List.htm.

The allergen used can be an allergen extract such as house-dust mite or pollen or fragments thereof containing at least one T-cell epitope or an entire or partial recombinant allergen protein such as Der p1 containing at least one T-cell epitope. The preferred route of administration is subcutaneous injection however, other routes such as nasal, oral or sublingual application can be effective as well. Another embodiment is the use of DNA vaccines that incorporate a gene encoding the entire or partial allergen sequence and containing at least one T-cell epitope sequence (Walker *et al.,* 2001).

An allergen vaccination course usually involves a build-up phase (increasing allergen dose) and a maintenance phase (maximum dosage of the allergen) in which the allergen is administered with a 1-2 month interval. The duration of allergen vaccination required to maintain improvement in clinical symptoms has been advised to 3 to 5 years of therapy (Bousquet *et al.,* 1998). Allergen vaccination is rarely started before the age of 5 years. When started early in the disease process, allergen vaccination may modify the progression of the disease.

Novel allergen vaccination strategies consist of the treatment with di-hydroxyvitamin D3 compounds at the time of allergen injection. These compound(s) may be co-injected subcutaneously together with the allergen or given separately by systemic (par)enteral administration.

The methods provided herein are suitable for treating any allergic disorders including, but not limited to rhinitis, food allergy, urticaria, atopic dermatitis and asthma.

**Table 1. Non exhausting list of inhibitors of NF-κB activation as described in literature, grouped as anti-oxidants, protease and protease inhibitors, IKβA phosphorylation and/or degradation inhibitors and miscellaneous inhibitors (modified from http://people.bu.edulgilmore/nf-kb).**

| **anti-oxidants** | **proteasome and/or protease inhibitors** | **IκBα phosphorylation and/or degradation inhibitors** | **miscellaneous inhibitors** |
|---|---|---|---|
| α-lipoic acid | ALLnL (N-acetyl- leucinyl-leucinyl- norleucinal, MG101) | Rocaglamides (Aglaia derivatives) | β-amyloid protein |
| α-tocopherol | Z-LLnV (carbobenzoxyl-leucinyl-leucinyl-norvalinal,MG 115) | Jesterone dimer | Glucocorticoids |
| Aged garlic extract | Z-LLL (carbobenzoxyl-leucinyl-leucinyl-leucinal, MG132) | Silibinin | IL-10 |
| Anetholdithiolthione (ADT) | Lactacystine, β-lactone | Quercetin | IL 13 |
| Butylated hydroxyanisole (BHA) | Boronic Acid Peptide | Staurosporine | IL-11 |
| Cepharanthine | Ubiquitin Ligase Inhibitors | Aspirin, sodium salicylate | Dioxin |
| Caffeic Acid Phenethyl Ester (3,4-dihydroxycinnamic acid, CAPE) | PS-341 | BAY-117821 (E3((4- methylphenyl)-sulfonyl)-2-propenenitrile) | Leptomycin B (LMB) |
| Catechol Derivatives | Cyclosporin A | BAY-117083 (E3((4-t- butylphenyl)- sulfonyl)-2-propenenitrile) | NLS Cell permeable peptides |
| Dibenzylbutyrol-actone lignans | FK506 (Tacrolimus) | Cycloepoxydon; 1- hydroxy-2- hydroxymethyl-3-pent-1-enylbenzene | o,o'-bismyristoyl thiamine disulfide (BMT) |
| Diethyldithio-carbamate (DDC) | Deoxyspergualin | Extensively oxidized low density lipoprotein (ox-LDL), 4-Hydroxynonenal (HNE) | ADP ribosylation inhibitors (nicotinamide, 3-aminobenzamide) |
| Diferoxamine | APNE (N-acetyl- DL-phenylalanine- b-naphthylester) | Ibuprofen | Atrial Natriuretic Peptide (ANP) |
| Dihydrolipoic Acid | BTEE (N-benzoyl L-tyrosine-ethylester) | Nitric Oxid (NO) | Atrovastat (HMG-CoA reductase inhibitor) |
| Disulfiram | DCIC (3,4-dichloroisocoumari n) | Prostaglandin Al | AvrA protein (Salmonella) |
| Dimethyldithio- carbamates (DMDTC) | DFP (diisopropyl fluorophosphate) | Sulfasalazine | Bovine serum albumin |
| Curcumin (Diferulolylmethane) | TPCK (N-a-tosyl- L-phenylalanine chloromethyl ketone) | YopJ (encoded by Yersinia pseudotuberculosis ) | Calcitriol (1α,25-dihydroxyvitamine D3) or analogs |
| Ebselen | TLCK(N-a-tosyl-L-lysine chloromethyl ketone) | A-melanocyte-stimulating hormone (α-MSH) | Capsiate |
| EPC-K1 (phosphodiester compound of vitamin E and vitamin C) | Phosphodiesterase inhibitors i.e. theophylline; pentoxyphylline | Aucubin | Catalposide |
| Epigallocatechin-3-gallate (EGCG; green tea polyphenols) | | β-lapachone | Clarithromycin |
| Ethylene Glycol Tetraacetic Acid (EGTA) | | Capsaicin (8-methyl-N-vanillyl-6-nonenamide) | Diamide |
| Gamma-glutamylcysteine synthetase (gamma-GCS) | | Core Protein of Hepatitis C virus (HCV) | E3330 (quinone derivative) |
| Glutathione | | Diamide (tyrosine phosphatase inhibitor) | Epoxyquinol A (fungal metabolite) |
| IRFI 042 | | E-73 (cycloheximide analog) | Glycyrrhizin |
| Iron tetrakis | | Emodin (3-methyl-1,6,8- trihydroxyanthraquinone) | Hematein (plant compound) |
| L-cysteine | | Erbstatin (tyrosine kinase inhibitor) | Herbimycin A |
| Lacidipine | | Estrogen (E2) | Hypericin |
| Magnolol | | Fungal gliotoxin | Hydroquinone (HQ) |
| Manganese Superoxide Dismutase (Mn-SOD) | | Genistein (tyrosine kinase inhibitor) | IL-4 |
| Melatonin | | IL-13 | IkB-like proteins (encoded by ASFV) |
| N-acetyl-L-cysteine (NAC) | | Leflunomide metabolite (A77 1726) | Kamebakaurin |
| Nordihydroguaiaritic acid (NDGA) | | Neurofibromatosis -2 (NF-2) protein | KT-90 (morphine synthetic derivative) |
| Ortho-phenanthroline | | Pervanadate (tyrosine phosphatase inhibitor) | Metals (chromium, cadmium, gold, lead, mercury, zinc, arsenic) |
| Phenylarsine oxide (PAO, tyrosine phosphatase inhibitor) | | Phenylarsine oxide (PAO, tyrosine phosphatase inhibitor) | Mevinolin, 5'-methylthioadenosi ne (MTA) |
| Pyrrolinedithiocarbamate (PDTC) | | Pituitary adenylate cyclase-activating polypeptide (PACAP) | N-ethyl-maleimide (NEM) |
| Quercetin | | Resiniferatoxin | Nicotine |
| Red wine | | Sesquiterpene lactones (parthenolide) | 1,2,3,4,6-penta-O-galloyl-beta-D-glucose |
| Rotenone | | Thiopental | Pentoxifylline (1-(5'-oxohexyl) 3,7-dimetylxanthine, PTX) |
| S-allyl-cysteine (SAC, garlic compound) | | Triglyceride-rich lipoproteins | Phenyl-N-tert-butylnitrone (PBN) |
| Tepoxaline (5-(4- chlorophenyl)-N- hydroxy-(4-methoxyphenyl)-N-methyl-1H-pyrazole-3-propanamide) | | Vasoactive intestinal peptide | Pyrithione |
| Vitamin C | | HIV-1 Vpu protein | Rolipram |
| Vitamin E derivatives | | Dibenzyl butyrolactone lignans | Quinadril (ACE inhibitor) |
| a-torphryl succinate | | Aurintricarboxylic acid | Ribavirin |
| a-torphryl acetate | | BAY 11-7082 | Secretory leukocyte protease inhibitor (SLPI) |
| PMC (2,2,5,7,8- pentamethyl-6- hydroxychromane) | | BAY 11-7085 | Serotonin derivative (N-(p-coumaroyl) serotonin, SC) |
| Carnosol | | IKK-NBD peptide | Silymarin |
| Sodium selenite | | Piceatannol | Sulfasalazine |
| Mol 294 | | | Vascular endothelial growth factor (VEGF) |
| | | | D609 (phosphatidylcholi ne-phospholipase C inhibitor) |
| | | | ethyl 2-[(3-methyl-2,5-dioxo(3-pyrrolinyl)) arnino]-4-(trifluoromethyl) pyrimidine-5-carboxylate |
| | | | Cycloprodigiosin hycrochloride |
| | | | RO31-8220 (PKC inhibitor) |
| | | | SB203580 (p38 MAPK inhibitor) |
| | | | Tranilast [N-(3,4-dimethoxycinnamo yl)anthranilic acid] |
| | | | Triptolide (PG490, extract of Chinese herb) |
| | | | LY294,002 |
| | | | Mesalamine |
| | | | Qingkailing and Shuanghuanglian (Chinese medicinal preparations) |
| | | | Tetrathio-molybdate |
| | | | Na⁺/H⁺ exchange inhibitors i.e. amiloride |
| | | | Gliotoxin |
| | | | Estriol |
| | | | Wortmannin (fungal metabolite) |
| | | | Inducers of heat-shock proteins, i.e. curcumin |
| | | | Helenalin |
| | | | |

The invention is further explained in more detail in the following description, which is not limiting the invention.

### EXPERIMENTAL PART

### MATERIALS AND METHODS

Animals. Animal care and use were performed in accordance with the guidelines of the Dutch Committee of Animal Experiments. Specific pathogenfree male BALB/c mice (5-6 wk old) were purchased from Charles River (Maastricht, The Netherlands) and housed in macrolon cages in a laminar flow cabinet and provided with food and water ad libitum.

**Sensitization, treatment and challenge.** Mice (6-8 wk old) were sensitized intraperitoneally (i.p.) on days 0 and 7 with 10 µg ovalbumin (OVA, grade V, Sigma-Aldrich) in 0.1 ml alum (Pierce, Rockford, Illinois). Two weeks after the last sensitization, the mice were divided in six groups. The sham-immunotherapy and the OVA-immunotherapy groups were treated with 3 s.c. injections of respectively 0.2 ml pyrogen-free saline (B. Braun, Melsungen, Germany) or 1 mg OVA in 0.2 ml pyrogen-free saline on alternate days. In three groups, OVA-immunotherapy was co-injected with 0.1 µg, 0.03 µg or 0.01 µg 1α,25-dihydroxyvitamin D3 (1α,25(OH)2 VitD3), a selective inhibitor of NF-Kb. One group was treated with sham-immunotherapy and combined with co-injection of 0.1 µg 1α,25(OH)2 VitD3. Ten days after treatment, mice were exposed to three OVA inhalation challenges (10 mg/ml in saline) for 20 min every third day.

In a second series of experiments, OVA-immunotherapy, as described above, was carried out using a sub-optimal amount of 100 µg OVA in saline for OVA-immunotherapy. OVA-immunotherapy was either given alone or in combination with 0.01 µg 1α,25(OH)2 VitD3. Sham-immunotherapy alone or in combination with 0.01 µg 1α,25(OH)2 VitD3 served as control groups.

**Measurement of airway responsiveness in vivo.** Airway responsiveness to methacholine was measured after treatment but before OVA challenge (pre measurement) and twenty-four hours after the last OVA challenge. Airway responsiveness was measured in conscious, unrestrained mice using barometric whole-body plethysmography by recording respiratory pressure curves (Buxco, EMKA Technologies, Paris, France) in response to inhaled methacholine (acetyl-β-methylcholine chloride, Sigma-Aldrich). Airway responsiveness was expressed in enhanced pause (Penh), as described in detail previously (Deurloo et al., 2001). Briefly, mice were placed in a whole-body chamber and basal readings were determined for 3 min. Aerosolized saline, followed by doubling concentrations of methacholine (ranging from 3.13-25 mg/ml in saline), were nebulized for 3 min, and readings were determined for 3 min after each nebulization.

**Determination of OVA-specific IgE levels in serum.** From each mouse, serum was obtained after treatment but before OVA challenge (pre measurement) by a small incision in the tail vein. After measurement of airway responsiveness in vivo, mice were sacrificed by i.p. injection of 1 ml 10% urethane in saline and were bled by cardiac puncture. Subsequently, serum was collected and stored at -70°C until analysis. Serum levels of OVA-specific IgE were measured by sandwich ELISA as described previously (Deurloo et al., 2001).

**Analysis of the cellular composition of the bronchoalveolar lavage fluid**. Bronchoalveolar lavage (BAL) was performed immediately after bleeding of the mice by lavage of the airways through a tracheal cannule 5 times with 1 ml saline (37°C). Cells in the BALF were centrifuged and resuspended in cold PBS. The total number of cells in the BAL was determined using a Bürker-Türk counting-chamber (Karl Hecht Assistent KG, Sondheim/Röhm, Germany). For differential BAL cell counts, cytospin preparations were made (15 x g, 5 min, 4 °C, Kendro Heraues Instruments, Asheville, North Carolina). Next, cells were fixed and stained with Diff-Quick (Dade A.G., Düdingen, Switzerland). Per cytospin, 200 cells were counted and differentiated into mononuclear cells, eosinophils, and neutrophils by standard morphology and staining characteristics.

**Statistical analysis.** All data are expressed as mean ± standard error of mean (SEM). The airway dose-response curves to methacholine were statistically analysed by a general linear model of repeated measurements followed by post-hoc comparison between groups. Data were log transformed before analysis to equalize variances in all groups. All other data were analysed using a Student's t test (2-tailed, homosedastic). Results were considered statistically significant at the p<0.05 level.

### RESULTS 1

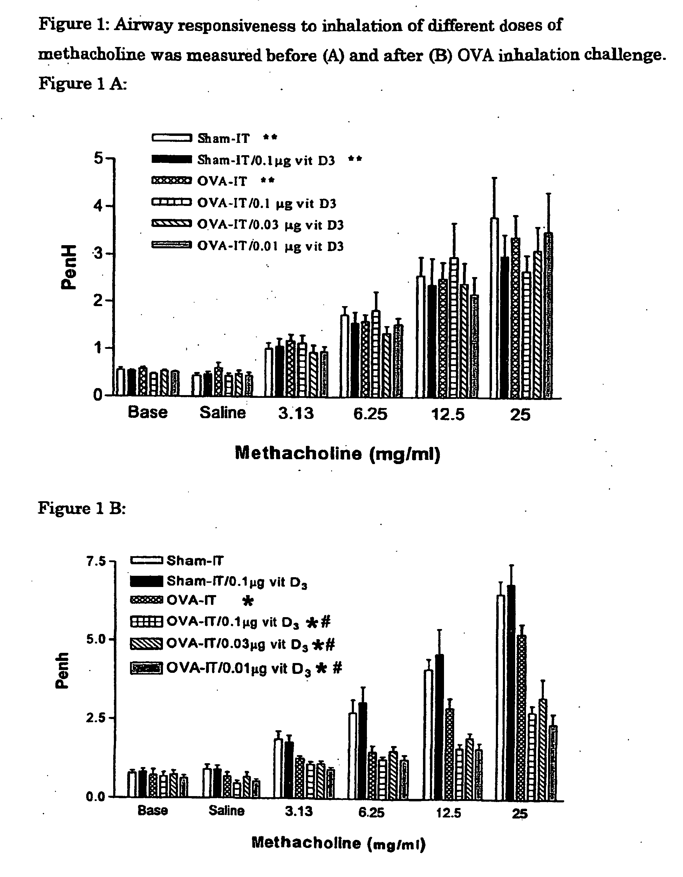

**Airway responsiveness in vivo.** No significant differences between all six groups were observed in airway responsiveness to methacholine after treatment but prior to OVA challenge (figure 1A). OVA sensitized BALB/c mice that received sham-immunotherapy displayed significant airway hyper-responsiveness (AHR) to methacholine after OVA inhalation challenge as compared to before challenge. Mice that received OVA-immunotherapy displayed significant AHR to methacholine after OVA challenge as compared to before challenge. However, OVA-immunotherapy partially reduced (P<0.05) AHR to methacholine as compared to sham-treated mice. Interestingly, co-injection of 1α,25(OH)2 VitD3, at all doses used, with OVA-immunotherapy significantly potentiated the reduction of AHR to methacholine compared to OVA-IT alone. Co-injection of 1α,25(OH)2 VitD3 with sham-immunotherapy did not affect AHR. It can be concluded that co-injection of the selective NF-κb inhibitor 1α,25(OH)2 VitD3 potentiates the suppressive effect of OVA-immunotherapy on AHR.

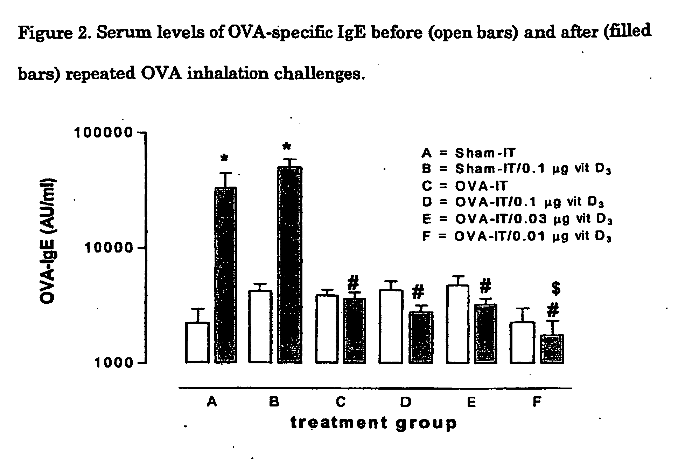

**OVA-specific IgE levels in serum.** OVA sensitized BALB/c mice that received sham-immunotherapy showed a significant increase in serum levels of OVA-specific IgE after OVA inhalation challenge as compared to before challenge (figure 2). In mice that received OVA-immunotherapy, serum OVA-specific IgE levels were significantly reduced after OVA challenge as compared to sham-treated OVA-challenged mice. Co-injection of 0.01 µg 1α,25(OH)2 VitD3 with OVA-immunotherapy significantly increased the reduction of serum IgE levels as compared to OVA-immunotherapy alone. Co-injection of 1α,25(OH)2 VitD3 with sham-immunotherapy did not affect serum IgE levels as compared to sham-immunotherapy alone. It can be concluded that co-injection of the selective NF-κb inhibitor 1α,25(OH)2 VitD3, at 0.01 µg, potentiates the suppression of serum OVA-specific IgE levels after OVA-immunotherapy.

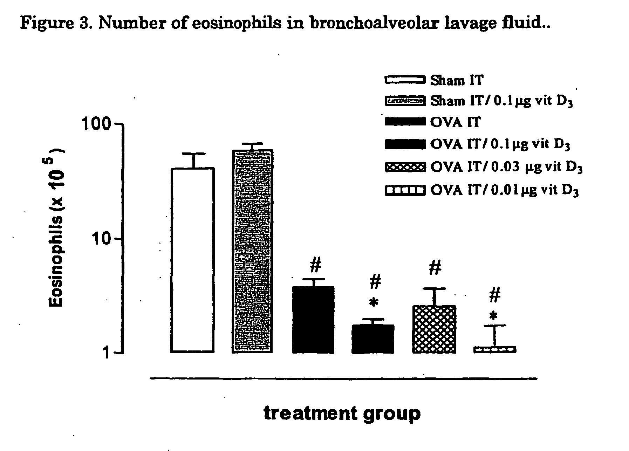

**Cellular composition of the bronchoalveolar lavage fluid.** In BALB/c mice, no eosinophils are present in BALF prior to OVA inhalation. OVA sensitized BALBIc mice that received sham-immunotherapy showed BALF eosinophilia after OVA inhalation challenge (figure 3). After OVA challenge of mice that received OVA-immunotherapy, the number of BALF eosinophils were significantly reduced as compared to sham-treated mice. Co-injection of 0.01 µg 1α,25(OH)2 VitD3 with OVA-immunotherapy significantly increased the reduction of BALF eosinophil numbers as compared to OVA-immunotherapy alone. Co-injection of 1α,25(OH)2 VitD3 with sham-immunotherapy did not significantly affect BAL eosinophil number. It can be concluded that co-injection of the selective NF-κb inhibitor 1α,25(OH)2 VitD3, at 0.01 µg, potentiates the suppression of BALF eosinophilia after OVA-immunotherapy.

Collectively, it is concluded that the selective NF-κb inhibitor 1α,25(OH)2 VitD3 is able to potentiate the beneficial effect of OVA-immunotherapy on airway hyper-responsiveness, serum IgE levels and airway eosinophilia, all important hallmarks of human asthma.

### RESULTS 2

In the results described above, the effect of co-injection of the selective NF-κB inhibitor 1α,25(OH)2 VitD3 with OVA-immunotherapy was most pronounced with regards to potentiation of the suppression of AHR. The potentiation of the suppression of BALF eosinophilia was less pronounced because the amount of OVA used for OVA-immunotherapy already induced a strong, almost maximal, suppression of BALF eosinophil numbers (Figure 3). Therefore, we examined the effect of co-injection of 0.01 µg 1α,25(OH)2 VitD3 with a sub-optimal amount of OVA (100 µg) for OVA-immunotherapy.

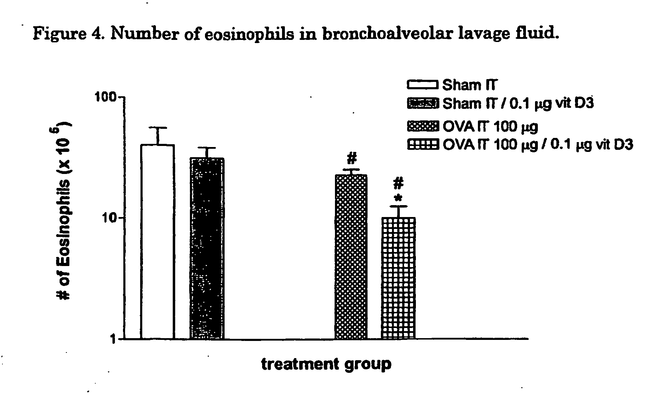

**Cellular composition of the bronchoalveolar lavage fluid.** In BALB/c mice, no eosinophils are present in BALF prior to OVA inhalation. OVA sensitized BALB/c mice that received sham-immunotherapy showed BALF eosinophilia after OVA inhalation challenge (figure 4). After OVA challenge of mice that received OVA-immunotherapy (100 µg) the number of BALF eosinophils were significantly reduced as compared to sham-treated mice. Co-injection of 0.01 µg 1α,25(OH)2 VitD3 with a sub-optimal dose of OVA-immunotherapy significantly increased the reduction of BALF eosinophil numbers as compared to OVA-immunotherapy alone. Co-injection of 1α,25(OH)2 VitD3 with sham-immunotherapy did not significantly affect BAL eosinophil number.

It can be concluded that co-injection of the selective NF-κb inhibitor 1α,25(OH)2 VitD3 with sub-optimal OVA-immunotherapy is able to potentiate the suppression of BAL eosinophil numbers by a sub-optimal dose of OVA. Furthermore, co-injection of the selective NF-κb inhibitor 1α,25(OH)2 VitD3 can reduce the amount of allergen (in this case OVA) needed to obtain a particular level of suppression by allergen-immunotherapy.

### DESCRIPTION OF FIGURES

Figure 1. Airway responsiveness to inhalation of different doses of methacholine was measured before (A) and after (B) OVA inhalation challenge. Ovalbumin sensitized BALB/c mice (n=6 per group) were treated with sham-immunotherapy (sham-IT) or OVA-IT alone or in combination with 0.1 µg, 0.03 µg or 0.01 µg 1α,25(OH)2 VitD3 (VitD3) prior to repeated OVA inhalation challenges. **:P<0.05 as compared to after OVA inhalation challenge; *: P<0.05 as compared to sham-treated mice; #: P<0.05 as compared to OVA-IT alone.
Figure 2. Serum levels of OVA-specific IgE before (open bars) and after (filled bars) repeated OVA inhalation challenges. *: P<0.05 as compared to before OVA inhalation challenges. #: P<0.05 as compared to sham-IT treated mice. $: P<0.05 as compared to OVA-IT alone.
Figure 3. Number of eosinophils in bronchoalveolar lavage fluid. #:P<0.05 as compared to sham-IT treated mice; *:P<0.05 as compared to OVA-IT alone.
Figure 4. Number of eosinophils in bronchoalveolar lavage fluid. #: P<0.05 as compared to sham-IT treated mice; *:P<0.05 as compared to OVA-IT alone.

### References

Baxter, A.G. & Hodgkin, P.D. (2002). Activation rules: the two-signal theories of immune activation. Nat Rev Immunol, 2, 439-46.
Bousquet, J., Lockey, R., Malling, H.J., Alvarez-Cuesta, E., Canonica, G.W., Chapman, M.D., Creticos, P.J., Dayer, J.M., Durham, S.R., Demoly, P., Goldstein, R.J., Ishikawa, T., Ito, K., Kraft, D., Lambert, P.H., Lowenstein, H., Muller, U., Norman, P.S., Reisman, R.E., Valenta, R., Valovirta, E. & Yssel, H. (1998). Allergen immunotherapy: therapeutic vaccines for allergic diseases. World Health Organization. American academy of Allergy, Asthma and Immunology. Ann Allergy Asthma Immunol, 81, 401-5.
Daynes, R.A. & Jones, D.C. (2002). Emerging roles of ppars in inflammation and immunity. Nat Rev Immunol, 2, 748-59.
Dhodapkar, M.V., Steinman, R.M., Krasovsky, J., Munz, C. & Bhardwaj, N. (2001). Antigen-specific Inhibition of Effector T Cell Function in Humans after Injection of Immature Dendritic Cells. J Exp Med, 193, 233-238.
Foletta, V.C., Segal, D.H. & Cohen, D.R. (1998). Transcriptional regulation in the immune system: all roads lead to AP-1. J Leukoc Biol, 63, 139-52.
Gallucci, S. & Matzinger, P. (2001). Danger signals: SOS to the immune system. Curr Opin Immunol, 13, 114-119.
Herlaar, E. & Brown, Z. (1999). p38 MAPK signalling cascades in inflammatory disease. Mol Med Today, 5, 439-47.
Hihi, A.K., Michalik, L. & Wahli, W. (2002). PPARs: transcriptional effectors of fatty acids and their derivatives. Cell Mol Life Sci, 59, 790-8.
Jenne, L., Schuler, G. & Steinkasserer, A. (2001). Viral vectors for dendritic cell-based immunotherapy. Trends Immunol, 22, 102-107.
Jonuleit, H., Schmitt, E., Steinbrink, K. & Enk, A.H. (2001). Dendritic cells as a tool to induce anergic and regulatory T cells. Trends Immunol, 22, 394-400. Li, Q. & Verma, I.M. (2002). NF-kappaB regulation in the immune system. Nat Rev Immunol, 2, 725-34.
Matzinger, P. (2002). The danger model: a renewed sense of self. Science, 296, 301-5.
McKay, L.I. & Cidlowski, J.A. (1999). Molecular control of immune/inflammatory responses: interactions between nuclear factor-kappa B and steroid receptor-signaling pathways. Endocr Rev, 20, 435-59.
Nencioni, A., Grunebach, F., Zobywlaski, A., Denzlinger, C., Brugger, W. & Brossart, P. (2002). Dendritic cell immunogenicity is regulated by peroxisome proliferator-activated receptor gamma. J Immunol, 169, 1228-35.
Pulendran, B., Palucka, K. & Banchereau, J. (2001). Sensing pathogens and tuning immune responses. Science, 293, 253-6.
Reis e Sousa, C. (2001). Dendritic cells as sensors of infection. Immunity, 14, 495-8.
Roncarolo, M.G., Levings, M.K. & Traversari, C. (2001). Differentiation of T Regulatory Cells by Immature Dendritic Cells. J Exp Med, 193, F5-F10. Walker, C. & Zuany-Amorim, C. (2001). New trends in immunotherapy to prevent atopic diseases. Trends Pharmacol Sci, 22, 84-90.

## Claims

1. A di-hydroxyvitamin D3 compound and an allergen for use in treating allergic disorders by inducing and/or increasing tolerance to said allergen.

2. Use according to claim 1, wherein said allergen is capable of inducing or sustaining an allergic disease.

3. Use according to claim 2, wherein said allergic disease is asthma.

4. Pharmaceutical composition for use in treating allergic disorders by inducing and/or increasing tolerance to an allergen comprising a di-hydroxyvitamin D3 compound, said composition further comprising said allergen and a suitable diluent.

5. Pharmaceutical composition for use according to claim 4, wherein said allergen is capable of inducing or sustaining an allergic disease.

6. Pharmaceutical composition for use according to claim 5, wherein said allergic disease is asthma.

## Patentansprüche

1. Dihydroxyvitamin-D3-Verbindung und ein Allergen zur Verwendung bei der Behandlung allergischer Störungen durch Induzieren und/oder Erhöhen der Toleranz gegen das Allergen.

2. Verwendung nach Anspruch 1, wobei das Allergen eine allergische Krankheit induzieren oder aufrechterhalten kann.

3. Verwendung nach Anspruch 2, wobei die allergische Krankheit Asthma ist.

4. Pharmazeutische Zusammensetzung zur Verwendung bei der Behandlung allergischer Störungen durch Induzieren und/oder Erhöhen der Toleranz gegen ein Allergen, die eine Dihydroxyvitamin-D3-Verbindung umfasst, wobei die Zusammensetzung ferner das Allergen und ein geeignetes Verdünnungsmittel umfasst.

5. Pharmazeutische Zusammensetzung zur Verwendung nach Anspruch 4, wobei das Allergen eine allergische Krankheit induzieren oder aufrechterhalten kann.

6. Pharmazeutische Zusammensetzung zur Verwendung nach Anspruch 5, wobei die allergische Krankheit Asthma ist.

## Revendications

1. Composé de dihydroxyvitamine D3 et allergène pour utilisation dans le traitement de troubles allergiques par induction et/ou augmentation d'une tolérance audit allergène.

2. Utilisation selon la revendication 1, dans laquelle ledit allergène est capable d'induire ou d'entretenir une maladie allergique.

3. Utilisation selon la revendication 2, dans laquelle ladite maladie allergique est l'asthme.

4. Composition pharmaceutique pour utilisation dans le traitement de troubles allergiques par induction et/ou augmentation d'une tolérance à un allergène, comprenant un composé de dihydroxyvitamine D3, ladite composition comprenant en outre ledit allergène et un diluant approprié.

5. Composition pharmaceutique pour utilisation selon la revendication 4, dans laquelle ledit allergène est capable d'induire ou d'entretenir une maladie allergique.

6. Composition pharmaceutique pour utilisation selon la revendication 5, dans laquelle ladite maladie allergique est l'asthme.
